Europäisches Patentamt

European Patent Office

Office européen des brevets

(19)

(11) Veröffentlichungsnummer: **0 064 278**
**B1**

(12) **EUROPÄISCHE PATENTSCHRIFT**

(45) Veröffentlichungstag der Patentschrift:
14.08.85

(51) Int. Cl.⁴: **A 61 F 2/18,** A 61 L 17/00

(21) Anmeldenummer: **82103648.0**

(22) Anmeldetag: **29.04.82**

(54) Ambossprothese und Verfahren zu ihrer Herstellung.

Verbunden mit 82901351.5/0077366 (europäische Anmeldenummer/Veröffentlichungsnummer) durch Entscheidung vom 27.03.84.

(30) Priorität: 29.04.81 DE 3117024
26.03.82 DE 3211211

(43) Veröffentlichungstag der Anmeldung:
10.11.82 Patentblatt 82/45

(45) Bekanntmachung des Hinweises auf die Patenterteilung:
14.08.85 Patentblatt 85/33

(84) Benannte Vertragsstaaten:
FR GB IT NL

(56) Entgegenhaltungen:
WO - A - 82/01127
DE - B - 2 451 969
FR - A - 2 230 598
FR - A - 2 243 915
FR - A - 2 297 887
FR - A - 2 327 758
US - A - 3 919 723
US - A - 4 052 754
US - A - 4 052 754

(73) Patentinhaber: **ERNST LEITZ WETZLAR GMBH,**
**Ernst-Leitz-Strasse 30 Postfach 20 20,**
**D-6330 Wetzlar 1 (DE)**

(72) Erfinder: **Reck, Ralf, Südring 189,**
**D-6500 Mainz-Bretzenheim (DE)**
Erfinder: **Brömer, Heinz, Hundsrück 7, D-6330 Wetzlar**
**(DE)**
Erfinder: **Deutscher, Klaus, Lerchenweg 20,**
**D-6330 Wetzlar (DE)**
Erfinder: **Franek, Henning, Mühlenkopfstrasse 5,**
**D-6333 Braunfels-Tiefenbach (DE)**

ACTORUM AG

### Beschreibung

Die Erfindung betrifft eine im Mittelohrbereich zwischen Steigbügel und Hammergriff bzw. zwischen Steigbügel und Hammergriff und Trommelfell implantierbare Ambossprothese.

Aufgrund pathologischer Veränderungen bzw. chirurgischer Eingriffe im Mittelohrbereich kann es erforderlich werden, zur Rekonstruktion der akustomechanischen Funktion der Gehörknöchelchen-Kette, zu der der Hammer (malleus), der Amboss (incus) und der Steigbügel (stapes) gehören, mindestens ein Gehörknöchelchen durch ein Implantat zu ersetzen.

Die vorliegende Anmeldung betrifft speziell eine Prothese für den teilweisen oder ganz entfernten Amboss, welche in der Paukenhöhle zwischen Hammer und Steigbügel implantiert werden kann.

Dabei besteht die Aufgabe darin, eine solche Prothese vorzusehen, die trotz des anatomisch bedingten geringen Abstandes zwischen dem Hammergriff und dem processus cochleariformis bzw. der Sehne des musculus tensor tympani eine den jeweiligen örtlichen anatomischen Raumverhältnissen optimal anpassbare Raumform aufweist, dass sie dauerhaft in situ positionierbar ist und dass sie die Schwingungen innerhalb der Gehörknöchelchen-Kette als künstliches Zwischenglied zwischen Trommelfell und Steigbügel reizlos weiterleitet. Der Erfindung liegt weiterhin die Aufgabe zugrunde, bei der vorgeschlagenen Ambossprothese diejenigen Oberflächenteilbereiche, die nicht für den eigentlichen Verbund mit dem Knochen (Verwachsungszone: Implantat/ knöchernes Hartgewebe) vorgesehen sind, derart auszubilden, dass sie biochemisch vollkommen resistent und bioinaktiv sind. Darüber hinaus besteht die Aufgabe darin, ein Verfahren zur nachträglichen Bioinaktivierung von ausgewählten Teilbereichen an sich bioaktiver Prothesen bzw. Prothesenteile anzugeben.

Die Aufgabe wird erfindungsgemäss dadurch gelöst, dass die Ambossprothese eine sich verjüngende, geschlossene geometrische Raumform aufweist, bestehend aus zwei als Basis- und Deckfläche ausgebildeten Abschlussflächen und einer diese verbindende Mantelfläche, wobei zusätzlich noch die Beziehungen gelten, dass die Basisfläche grösser als die Deckfläche, der grösste Durchmesser der Basisfläche kleiner als die Distanz zwischen beiden Abschlussflächen und der grösste Durchmesser der Deckfläche grösser als der grösste Halbmesser der Basisfläche ist und dass die Ambossprothese aus bioaktivem Material besteht. Dabei weisen beide Abschlussflächen zweckmässigerweise analoge geometrische Formen, wie Kreis, Ellipse, regelmässiges Vieleck, unregelmässiger Polygonzug bzw. Kombinationen davon, auf. Es ist aber auch möglich, dass die geometrischen Formen beider Abschlussflächen nicht analog sind, sondern dass beispielsweise die Basisfläche einerseits eine modifizierte Kreisfläche darstellt, von der zwei Kreisabschnitte abgetrennt sind, und die Deckfläche andererseits eine vollständige Kreis- oder Ellipsenfläche darstellt.

Es ist möglich, dass beide Abschlussflächen in zueinander parallel verlaufenden Ebenen liegen und dass die Verbindungslinie der geometrischen Mittelpunkte beider Abschlussflächen auf diesen senkrecht steht.

Nach einer besonderen Ausführungsform der vorliegenden Erfindung besteht die Raumform aus einem Kegelstumpf, von dem längs seiner Mantelfläche zwei paraboloidförmige symmetrische Abschnitte entfernt wurden derart, dass die Konturen der dabei entstandenen Schnittflächen jeweils die Form einer auf der Spur der Deckfläche aufsitzenden und sich zur Basisfläche öffnenden Parabel aufweist.

Vorteilhafterweise beträgt der grösste Durchmesser der Basisfläche 2,3 bis 3,0 mm – vorzugsweise 2,6 mm –, der grösste Durchmesser der Deckfläche 1,5 bis 2,0 mm – vorzugsweise 1,7 mm – und die Distanz zwischen beiden Abschlussflächen 3,0 bis 4,8 mm – vorzugsweise 4,0 mm –. Die erfindungsgemässe Prothese kann zusätzlich – vorzugsweise im Bereich ihrer beiden Abschlussflächen – mindestens eine Ausnehmung, beispielsweise eine Mulde, eine Abschrägung, eine Rille usw. aufweisen. Nach einer bevorzugten Ausführungsform ist das bioaktive Material, aus dem die Ambossprothese besteht, eine Bioglaskeramik. Als weitere bioaktive Materialien können alternativ apatithaltige Sinterprodukte oder andere bioaktive Verbundmaterialien verwendet werden. Auch ist es prinzipiell möglich, die vorgeschlagene Prothese aus einem – beispielsweise bioinerten – Kernmaterial zu fertigen, das eine Beschichtung mit bioaktiven Substanzen aufweist.

Die Aufgabe wird bei einer Prothese der eingangs genannten Art weiterhin dadurch gelöst, dass sie zusätzlich in mindestens einem Oberflächen-Teilbereich unlösliches bioinertes Material enthält bzw. partiell eine Beschichtung aus unlöslichem bioinertem Material aufweist. Dabei kann die Beschichtung aus mindestens einer additiv aufgebrachten Schutzschicht mit einer Dicke zwischen 0,25 und 10 μm oder aus mindestens einer subtraktiv erzeugten Konversionsschicht mit einer Dicke zwischen 0,25 und 5 μm bestehen. Es ist auch möglich, dass die Beschichtung aus mindestens einer prothesenkernseitig erzeugten Konversionsschicht und mindestens einer auf dieser aufliegenden, additiv aufgebrachten Schutzschicht besteht. Mit Vorteil besteht die Prothese zumindest in ihrem Mantelbereich aus unlöslichem bioinertem Material. Es ist indes auch möglich, eine beschichtete Prothese vorzusehen, bei der lediglich die Abschlussflächen – also die Basisfläche und die Deckfläche – aus bioaktivem Material bestehen.

Das bioinerte Material bzw. die bioinerte Schicht kann aus mindestens einer der folgenden Substanzen bestehen: Metalle, wie Gold, Platin, Titan, sowie Metallegierungen; Kohlenstoff in geeigneten Modifikationen, wie pyrolytischer Kohlenstoff (Graphit); Kohlenstoffverbindungen, wie Siliziumkarbid (SiC), Titankarbid (TiC), Borkarbid

(B$_4$C); Sonderkeramische Werkstoffe, wie hexagonales Bornitrid (BN), Titannitrid (TN), Siliziumnitrid (Si$_3$N$_4$); teilkristalline anorganische Verbundsysteme, wie Emails; anorganische Einkomponenten- (z. B. Kieselglas) oder Mehrkomponenten-Gläser; Oxide, wie Titandioxid (TiO$_2$), Zirkondioxid (ZrO$_2$) und Aluminiumoxid (Al$_2$O$_3$). Nach einer vorteilhaften Ausführungsform besteht das bioinerte Material bzw. die bioinerte Schicht aus einer(m) apatitfreien Residual-Bioglaskeramik bzw. -Biokeramik bzw. Bioglas, welche(s) gegebenenfalls zusätzlich eine Silan-Schicht aufweist.

Nach dem erfindungsgemässen Verfahren wird die Mantelfläche dieser Prothese unter gezielter Stoffzufuhr einer Oberflächen-Nachbehandlung zur Erzeugung mindestens einer additif aufgebrachten, dauerhaft festsitzenden, anorganischen, in vivo als biochemische Sperrschicht wirkenden, bioinerten Schutzschicht unterworfen. Es ist aber ebenso möglich, ein Beschichtungsverfahren vorzusehen, bei dem die Mantelfläche dieser Prothese unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions-(Auslaugungs-)Schicht unterworfen wird. Die additive Schutzschicht kann vorzugsweise durch mindestens einen der folgenden Verfahrensschritte aufgebracht werden: Galvanisieren oder Bedampfen; Aufsputtern; Abscheiden aus organischen Lösungen oder Aufdampfen im Vakuum; Tauchen, Sprühen oder Aufstreuen mit nachfolgender thermischer Behandlung; Eintauchen in Wasserglas mit anschliessendem Aufheizen auf ca. 400° C bzw. Tauchglasieren aus ein- oder mehrkomponentigen Schmelzgemengen; Simultan-Aufdampfen oder Aufdampfen der Metalle mit nachfolgender Oxidierungsbehandlung.

Die subtraktive Schutzschicht kann gemäss der vorliegenden Erfindung erhalten werden, indem die zu beschichtende Mantelfläche mit wässrigen, sauren Lösungen oder mit wässrigen Salzlösungen mit Normalitäten zwischen 0,001 und 0,1 zwischen 5 Minuten und 3 Stunden bei Temperaturen zwischen 20 bis 100° C behandelt wird. Dabei kann als wässrige, saure Lösung 0,1–0,001 normale Salzsäure (HCl) oder als wässrige Salzlösung 0,001 bis 0,25 normale Standard-Azetat-Puffer-Lösung verwendet werden. Schliesslich ist es möglich, die beschichteten Teile der Prothese anschliessend einer thermischen Versiegelungs- und/oder Silanisierungsbehandlung zu unterwerfen.

In den Zeichnungen sind Ausführungsbeispiele der Erfindung schematisch dargestellt. Es zeigen:

Fig. 1 einen Schnitt durch den Mittelohrtrakt mit implantierter Prothese;

Fig. 2a und 2b zwei perspektivische Ansichten der erfindungsgemässen Prothese;

Fig. 3 die in Fig. 1 dargestellte Prothese, welche im Mantelbereich eine additive Schutzschicht aufweist;

Fig. 4 die in Fig. 1 dargestellte Prothese, welche in ihrem Mantelbereich eine subtraktive Schutzschicht aufweist;

Fig. 5 eine perspektivische Ansicht gemäss Fig. 2b der erfindungsgemässen Ambossprothese mit partieller additiver Beschichtung.

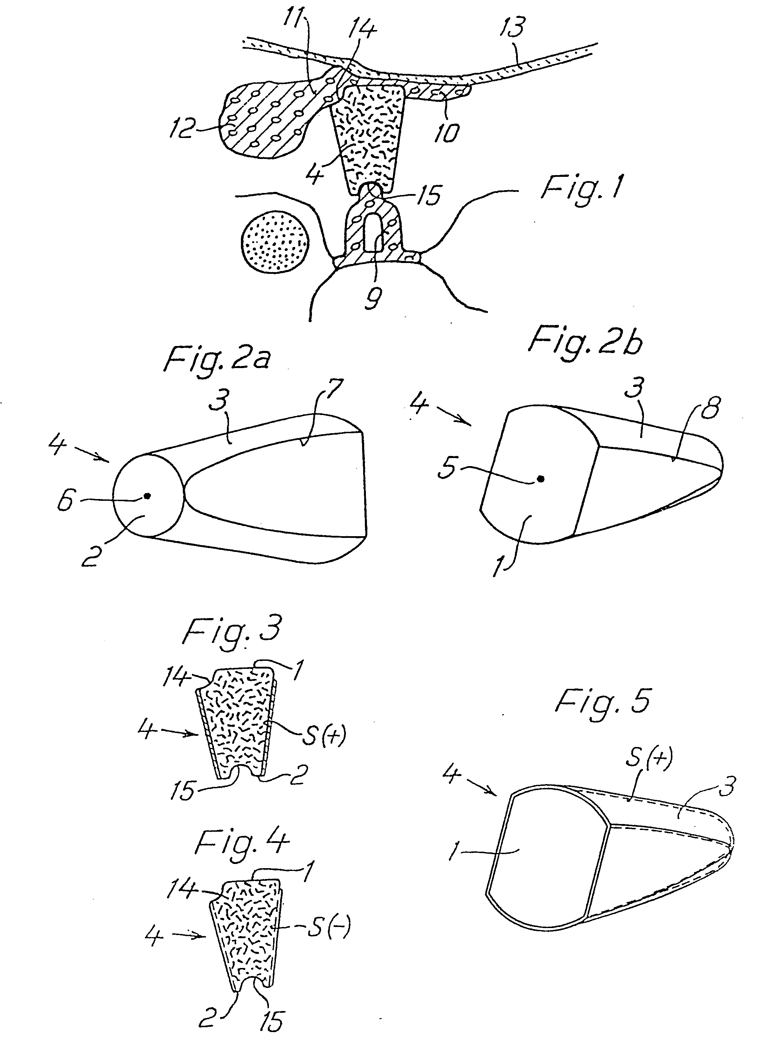

In Fig. 1 sind als natürliche Gehörknöchelchen der Steigbügel 9 und der Hammer 11, dessen Griff 10 am Trommelfell 13 anliegt, vorhanden. Es fehlt der Amboss, der normalerweise einen Kontakt zwischen dem Hammerkopf 12 und der Bügelpartie des Steigbügels 9 herstellt. Die erfindungsgemässe Ambossprothese 4 überbrückt die defekte Verbindung zwischen dem Steigbügel 9 und dem Hammer 11, genauer: dem Hammergriff 10.

Wegen der individuellen anatomischen Situation am Implantationsort ist es erforderlich, die Prothese 4 – insbesondere im Bereich ihrer beiden Abschlussflächen 1 bzw. 2 – mit nachträglich anzubringenden Ausnehmungen, beispielsweise mit einer Mulde 14, einer Abschrägung, einer Rille 15 usw. zu versehen.

Es ist natürlich auch möglich, mindestens eine Ausnehmung, beispielsweise eine schwach konturierte Rille, von vorne herein an der Ambossprothese vorzusehen, so dass sie während des Implantationsvorgangs im Bedarfsfall lediglich etwas nachgearbeitet werden muss, um passgenau positioniert werden zu können. Das verwendete Biomaterial, insbesondere auch die bioaktive Glaskeramik, lässt alle bekannten Materialabtragungsverfahren und -behandlungsverfahren, wie Fräsen, Schleifen, Bohren, Schneiden, Sägen, Schmirgeln, Polieren usw. zu.

In den Fig. 2a und 2b ist die Raumform einer erfindungsgemässen Ambossprothese 4 in zwei perspektivischen Ansichten dargestellt. In Fig. 2a erkennt man die Deckfläche 2, die im dargestellten Fall als Kreis mit dem Mittelpunkt 6 ausgebildet ist. In Fig. 2b ist die Basisfläche 1 mit dem Mittelpunkt 5 zu erkennen. Sie stellt eine Kreisfläche dar, von der zwei gleichgrosse und diametral gegenüberliegende Kreisabschnitte abgetrennt wurden. Beide Abschlussflächen 1 und 2 liegen in zueinander parallelen Ebenen. Die Distanz zwischen beiden entspricht der Länge der die Mittelpunkte 5 und 6 verbindenden Geraden, die gleichzeitig die Symmetrieachse dieser Ausführungsform der erfindungsgemässen Prothese darstellt. Natürlich ist es auch möglich, dass die Ebenen beider Abschlussflächen 1 und 2 nicht parallel verlaufen, so dass die die Mittelpunkte 5 und 6 verbindende Gerade auf wenigstens einer der Abschlussflächen 1 und 2 nicht senkrecht steht. Schliesslich ist auch eine abgewandelte Raumform möglich, bei der beide Abschlussflächen 1 und 2 zwar in zueinander parallelen Ebenen liegen, bei der aber die Verbindungslinie beider Mittelpunkte auf keiner der Abschlussflächen 1 und 2 senkrecht steht.

In den Fig. 2a und 2b stellt die die Abschlussflächen verbindende Mantelfläche 3 einen Kegelstumpfmantel dar, von welchem zwei zueinander symmetrische Abschnitte entfernt wurden. Die längs der gesamten Mantelfläche verlaufenden Schnittkonturen beider Abschnitte weisen jeweils die Form einer Parabel 7 bzw. 8 auf, die sich zur Basisfläche 1 hin öffnen und deren Scheitelpunkte die Begrenzungslinie der Deckfläche 2 tangieren.

Wie aus Fig. 1 ersichtlich wird, ist die Ambossprothese 4 am Implantationsort jeweils so orientiert, dass ihre Basisfläche 1 zum Hammergriff 10 und somit ihre Deckfläche 2 zum Steigbügel 9 gerichtet ist.

Eine bioaktive Glaskeramik ist in der DE-PS 2326100 ausführlich beschrieben. Andere geeignete Werkstoffe auf der Basis apatithaltiger Sinterprodukte sind aus den DE-PS 2346739 und DE-PS 2434979 bekannt. Polymere bioaktive Verbundmaterialien sind durch die DE-PS 2501683 geschützt. Die Beschichtung eines Kernimplantates mit bioaktivem Material geht beispielsweise aus der AT-PS 347023 hervor.

Der Einsatz des Biomaterials als Werkstoff für die erfindungsgemässe Prothese bietet gerade im Bereich der Mittelohrchirurgie besondere Vorteile, die in der DE-A-3036245.9 ausführlich beschrieben wurden.

Es ist bekannt, dass bioaktive Knochenersatzmaterialien – insbesonder Bioglas oder Bioglaskeramik – eine gewisse Oberflächenlöslichkeit aufweisen, welche vermutlich eine für das Zustandekommen des Knochen/Implantat-Verbundes notwendige Voraussetzung darstellt. Andererseits kann diese spezielle Eigenschaft unter besonders ungünstigen lokalanatomischen Bedingungen, wie sie insbesondere bei der Implantation einer Prothese im Weichgewebelager bzw. bei Kontaktierung einer derartigen Prothese mit Weichgewebe gegeben sind, dazu führen, dass die Langzeitstabilität des Implantates beeinträchtigt wird. Da die Eigenschaft die Bioaktivität durch besondere chemische Zusammensetzung des Implantationsmaterials erzielt wird, ist ein solches Material für die Implantation in andere Gewebe nicht besonders vorteilhaft. So kann z. B. beobachtet werden, dass die Implantation von bioaktivem Material stärkere empfindliche Reaktionen im Weichgewebe auslöst als ein bioinertes Material.

Wie aus Fig. 1 zu erkennen, berührt die Ambossprothese 4 in situ nur im Bereich der Basisfläche 1 und der Deckfläche 2 knöchernes Gewebe (10, 11; 9), während sie im Bereich ihrer Mantelfläche 3 mit nicht dargestelltem Weichgewebe in Berührung kommt. Es ist daher von Vorteil, in diesem Bereich eine die Mantelfläche 3 umhüllende bioinerte, unlösliche Schutzschicht vorzusehen. In Fig. 3 ist eine derartige Schicht – und zwar eine additive Schutzschicht S(+) – dargestellt. Im Bereich der Deckfläche 2 kann diese Schutzschicht S(+) partiell auch vorhanden sein, insbesondere dann, wenn die als Rille 15 bezeichnete Ausnehmung geometrisch so klein bemessen ist, dass noch Teilbereiche der Deckfläche 2 existieren, die nicht in direktem Kontakt zum Stapes stehen.

In Fig. 4 ist die Prothese 4 mit einer geometrisch nicht auftragenden, bioinerten, unlöslichen, subtraktiven Schutzschicht S(−) versehen. Die Fig. 5 gibt die mit einer additiven Beschichtung S(+) ihrer Mantelfläche 3 versehene Prothese 4 in perspektivischer Darstellung wieder.

Beide – die additive und die subtraktive – Schutzschichten S(+) und S(−) sind hinsichtlich ihrer Funktion gleichwirkend. Sie stellen gewissermassen biochemische «Korrosions»-Schutzschichten dar, die komplette Transport- und Durchlass-Barrieren für jedweden Stoff-(Ionen)-Austausch zwischen den chemischen Bestandteilen des Implantates und den Bestandteilen der physiologisch-biochemischen Körperflüssigkeiten gewährleisten.

Beispielsweise kann eine subtraktive Schutzschicht S(−) in vitro dadurch erhalten werden, dass bei einer aus bioglaskeramischem Voll-Material oder aus Bioglas-Material bestehenden Ambossprothese diejenigen Oberflächen-Teilbereiche, die zwangsweise – also aufgrund anatomischer Gegebenheiten – oder vom Otochirurgen gewollt – also beispielsweise bei dem Auskleiden bestimmter Implantatbereiche mit Epithelgewebe – mit Weichgewebe in Dauerkontakt kommen, chemisch in der Weise vorbehandelt werden, dass wässrige saure Lösungen und/oder wässrige Salzlösungen in Normalitäten zwischen 0,001 und 0,1 die ursprüngliche bioglaskeramische Oberfläche attackieren, wobei Angriffs-(Auflösungs- bzw. Auslaugungs-) und Austausch-Reaktionen nebeneinander ablaufen mit der Folge, dass es zunächst zu einer Verarmung und schliesslich zu einer vollständigen Zerstörung (Umwandlung) einer Phase – insbesondere der kristallinen Komponente(n) – des glaskeramischen Verbundsystems kommt. Zum chemischen Angriff können aber auch Basen und Puffersysteme verwendet werden, die in Abhängigkeit von ihrem konkreten Chemismus, ihrer Konzentration und ihrem $P_H$-Wert gezielt auf bestimmte Phasenkomponenten des Bioglaskeramik-«Verbundsystems» einwirken und so die gewünschte Eigenschaftsänderung der Implantatoberfläche bewirken.

Die nach dieser kombinierten chemischen Behandlung verbleibende Glaskeramik, die ihrer Apatitanteile beraubt ist, sei mit «Residual-Bioglaskeramik» bezeichnet. Sie ist bezüglich ihrer chemischen Eigenschaften unlöslich, porenfrei und stoppt jeglichen Ionentransport; sie ist bezüglich ihrer biochemisch-physiologischen Wirkung bioinaktiv, also bio-«inert», und bezüglich ihrer mechanischen Eigenschaften abriebresistent und auf dem Prothesenkernmaterial festhaftend. Wie aus Fig. 4 ersichtlich, wird durch die Erzeugung einer subtraktiven Schicht S(−) das Gesamtvolumen des Implantates nicht verändert. Es finden vielmehr chemische Austausch- bzw- Umwandlungsvorgänge in den Oberflächenbereichen statt, die gewissermassen «nach innen» – also zum Prothesenkern hin – gerichtet sind. Dagegen befindet sich die in Fig. 3 dargestellte additive Schicht S(+) auf der Prothesenmantelfläche 3, so dass das Gesamtvolumen der beschichteten Prothese 4 (Fig. 3 bzw. Fig. 5) etwas vergrössert wurde.

Im Bedarfsfall kann die subtraktive Schicht S(−) zusätzlich auf thermischem Wege verdichtet bzw. versiegelt werden. Darüber hinaus ist auch eine Silanisierungsschicht zusätzlich aufbringbar.

## Patentansprüche

1. Zwischen Steigbügel und Hammergriff bzw. zwischen Steigbügel und Hammergriff und Trommelfell einbringbare Ambossprothese, dadurch gekennzeichnet, dass die Ambossprothese

a) eine sich verjüngende, geschlossene geometrische Raumform (4) aufweist, bestehend aus zwei als Basisfläche (1) und Deckfläche (2) ausgebildeten Abschlussflächen und einer diese verbindenden Mantelfläche (3), wobei zusätzlich die folgenden Beziehungen gelten:

α) die Basisfläche (1) ist grösser als die Deckfläche (2),

β) der grösste Durchmesser der Basisfläche (1) ist kleiner als die Distanz zwischen beiden Abschlussflächen,

γ) der grösste Durchmesser der Deckfläche (2) ist grösser als der grösste Halbmesser der Basisfläche (1); und

b) aus bioaktivem Material besteht.

2. Ambossprothese nach Anspruch 1, dadurch gekennzeichnet, dass beide Abschlussflächen (1 bzw. 2) analoge geometrische Formen, wie Kreis, Ellipse, regelmässiges Vieleck, unregelmässiger Polygonzug, bzw. Kombinationen davon, aufweisen.

3. Ambossprothese nach mindestens einem der vorherigen Ansprüche, dadurch gekennzeichnet, dass die geometrischen Formen beider Abschlussflächen (1 bzw. 2) nicht analog sind.

4. Ambossprothese nach einem der vorherigen Ansprüche, dadurch gekennzeichnet, dass beide Abschlussflächen (1 bzw. 2) in zueinander parallel verlaufenden Ebenen liegen.

5. Ambossprothese nach einem der vorherigen Ansprüche, dadurch gekennzeichnet, dass die Verbindungslinie der geometrischen Mittelpunkte (5 bzw. 6) beider Abschlussflächen (1 bzw. 2) auf diesen senkrecht steht.

6. Ambossprothese nach einem der Ansprüche 1 und 3 bis 5, dadurch gekennzeichnet, dass die Raumform (4) aus einem Kegelstumpf besteht, von dem längs seiner Mantelfläche (3) zwei paraboloidförmige symmetrische Abschnitte entfernt wurden derart, dass die Konturen der dabei entstandenen Schnittflächen jeweils die Form einer auf der Spur der Deckfläche (2) aufsitzenden und sich zur Basisfläche (3) öffnenden Parabel (7 bzw. 8) aufweist.

7. Ambossprothese nach einem der vorherigen Ansprüche, dadurch gekennzeichnet, dass der grösste Durchmesser der Basisfläche (1) 2,3 bis 3,0 mm – vorzugsweise 2,6 mm –, der grösste Durchmesser der Deckfläche (2) 1,5 bis 2,0 mm – vorzugsweise 1,7 mm – und die Distanz zwischen beiden Abschlussflächen (1 bzw. 2) 3,0 bis 4,8 mm – vorzugsweise 4,0 mm – beträgt.

8. Ambossprothese nach einem der vorherigen Ansprüche, dadurch gekennzeichnet, dass sie zusätzlich – vorzugsweise im Bereich ihrer beiden Abschlussflächen (1 bzw. 2) – mindestens eine Ausnehmung, beispielsweise eine Mulde (14), eine Abschrägung, eine Rille (15) usw., aufweist.

9. Ambossprothese nach Anspruch 1, dadurch gekennzeichnet, dass das bioaktive Material ein Verbundmaterial ist.

10. Ambossprothese nach Anspruch 1, dadurch gekennzeichnet, dass das bioaktive Material aus apatithaltigen Sinterprodukten besteht.

11. Ambossprothese nach Anspruch 1, dadurch gekennzeichnet, dass das bioaktive Material aus Glaskeramik besteht.

12. Ambossprothese nach Anspruch 1, dadurch gekennzeichnet, dass sie aus einem Kernmaterial mit einer Beschichtung aus bioaktivem Material besteht.

13. Ambossprothese nach mindestens einem der vorhergehenden Ansprüche, dadurch gekennzeichnet, dass sie zusätzlich in mindestens einem Oberflächen-Teilbereich unlösliches bioinertes Material enthält.

14. Ambossprothese nach Anspruch 13, dadurch gekennzeichnet, dass sie partiell eine Beschichtung aus unlöslichem bioinerten Material aufweist.

15. Ambossprothese nach Anspruch 14, dadurch gekennzeichnet, dass die Beschichtung aus mindestens einer additiv aufgebrachten Schutzschicht (S(+)) mit einer Dicke zwischen 0,25 und 10 μm besteht.

16. Ambossprothese nach Anspruch 14, dadurch gekennzeichnet, dass die Beschichtung aus mindestens einer subtraktiv erzeugten Konversionsschicht (S(−)) mit einer Dicke zwischen 0,25 und 5 μm besteht.

17. Ambossprothese nach den Ansprüchen 14 bis 16, dadurch gekennzeichnet, dass die Beschichtung aus mindestens einer prothesenkernseitig erzeugten Konversionsschicht (S(−)) und mindestens einer auf dieser aufliegenden, additiv aufgebrachten Schutzschicht (S(+)) besteht.

18. Ambossprothese nach mindestens einem der Ansprüche 13 bis 17, dadurch gekennzeichnet, dass sie zumindest in ihrem Mantelbereich (3) aus unlöslichem bioinerten Material besteht.

19. Ambossprothese nach mindestens einem der Ansprüche 13 bis 18, daurch gekennzeichnet, dass lediglich die Abschlussflächen: Basisfläche (1) und Deckfläche (2) aus bioaktivem Material bestehen.

20. Ambossprothese nach mindestens einem der Ansprüche 13 bis 19, dadurch gekennzeichnet, dass das bioinerte Material bzw. die bioinerte Schicht (S(+) bzw. S(−)) aus mindestens einer der folgenden Substanzen besteht:

a) Metalle, wie Gold, Platin, Titan sowie Metallegierungen;

b) Kohlenstoff in geeigneten Modifikationen, wie pyrolytischer Kohlenstoff (Graphit); Kohlenstoffverbindungen, wie Siliziumkarbid (SiC), Titankarbid (TiC), Borkarbid ($B_4C$);

c) Sonderkeramische Werkstoffe, wie hexagonales Bornitrid (BN), Titannitrid (TIN), Siliciumnitrid ($Si_3N_4$);

d) teilkristalline anorganische Verbundsysteme, wie Emails;

e) anorganische Einkomponenten- (z.B. Kieselglas) oder Mehrkomponenten-Gläser;

f) Oxide, wie Titandioxid (TiO$_2$), Zirkondioxid (ZrO$_2$) und Aluminiumoxid (Al$_2$O$_3$).

21. Ambossprothese nach mindestens einem der Ansprüche 13 bis 20, dadurch gekennzeichnet, dass das bioinerte Material bzw. die bioinerte Schicht (S(−)) aus einer(m) apatitfreien Residual-Bioglaskeramik bzw. -Biokeramik bzw. Bioglas besteht, welche(s) gegebenenfalls zusätzlich eine Silan-Schicht aufweist.

22. Verfahren zur Herstellung einer Ambossprothese nach mindestens einem der Ansprüche 13 bis 21, dadurch gekennzeichnet, dass die Mantelfläche (3) dieser Ambossprothese (4) unter gezielter Stoffzufuhr einer Oberflächen-Nachbehandlung zur Erzeugung mindestens einer additiv aufgebrachten, dauerhaft festsitzenden, anorganischen, in vivo als biochemische Sperrschicht wirkenden, bioinerten Schutzschicht (S(+)) unterworfen wird.

23. Verfahren zur Herstellung einer Ambossprothese nach mindestens einem der Ansprüche 13 bis 21, dadurch gekennzeichnet, dass die Mantelfläche (3) dieser Ambossprothese (4) unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions-(Auslaugungs-)Schicht (S(−)) unterworfen wird.

24. Verfahren nach Anspruch 22, dadurch gekennzeichnet, dass die Schutzschicht (S(+)) durch mindestens einen der folgenden Schritte vorzugsweise aufgebracht wird:

a) Galvanisieren oder Bedampfen;

b) Aufsputtern;

c) Abscheiden aus organischen Lösungen oder Aufdampfen im Vakuum;

d) Tauchen, Sprühen oder Aufstreuen mit nachfolgender thermischer Behandlung;

e) Eintauchen in Wasserglas mit anschliessendem Aufheizen auf ca. 400° C bzw. Tauchglasieren aus ein- oder mehrkomponentigen Schmelzgemengen;

f) Simultan-Aufdampfen oder Aufdampfen der Metalle mit nachfolgender Oxidierungsbehandlung.

25. Verfahren nach Anspruch 23, dadurch gekennzeichnet, dass die zu beschichtende Mantelfläche (3) mit wässrigen, sauren Lösungen oder mit wässrigen Salzlösungen mit Normalitäten zwischen 0,001 und 0,1 zwischen 5 Minuten und 3 Stunden bei Temperaturen zwischen 20 bis 100° C behandelt wird.

26. Verfahren nach Anspruch 25, dadurch gekennzeichnet, dass als wässrige, saure Lösung 0,1 bis 0,001 normale Salzsäure (HCl) verwendet wird.

27. Verfahren nach Anspruch 25, dadurch gekennzeichnet, dass als wässrige Salzlösung 0,001 bis 0,25 normale Standard-Azetat-Puffer-Lösung verwendet wird.

28. Verfahren nach Anspruch 23, dadurch gekennzeichnet, dass die beschichteten Teile (3) der Ambossprothese (4) anschliessend einer thermischen Versiegelungs- und/oder Silanisierungsbehandlung unterworfen werden.

**Claims**

1. Anvil prothesis introduceable between stirrup and hammer or between stirrup and hammer and ear drum, characterised thereby, that the anvil prothesis

a) displays a narrowing, compact geometrical spatial shape (4) consisting of two end surfaces formes as base surface (1) and cover surface (2) and of a shell surface (3) connecting these, wherein the following relationships apply additionally:

α) the base surface (1) is greater than the cover surface (2),

β) the greatest diameter of the base surface (1) is smaller than the distance between both the end surfaces,

γ) the greatest diameter of the cover surface (2) is greater than the greatest radius of the base surface (1) and

b) consists of bio-active material.

2. Anvil prothesis according to claim 1, characterised thereby, that both end surfaces (1 or 2) display analogous geometrical shapes, such as circle, ellipse, regular polygon, irregular polygon course or combinations thereof.

3. Anvil prothesis according to at least one of the preceding claims, characterised thereby, that the geometric shapes of both the end surfaces (1 or 2) are not analogous.

4. Anvil prothesis according to one of the preceding claims, characterised thereby, that both end surfaces (1 or 2) lie in planes extending parallelly each to the other.

5. Anvil prothesis according to one of the preceding claims, characterised thereby, that the line connecting the geometric centres (5 or 6) of both end surfaces (1 or 2) is perpendicular to these.

6. Anvil prothesis according to one of the claims 1 and 3 to 5, characterised thereby, that the spatial shape (4) consists of a conical frustrum, from which two symmetrical paraboloid portions were removed along its shell surface (3) in such a manner that the outlines of the surfaces of section, that have arisen in that case, each display the shape of a parabola (7 or 8) sitting on the track of the cover surface (2) and opening towards the base surface (3).

7. Anvil prothesis according to one of the preceding claims, characterised thereby, that the greatest diameter of the base surface (1) amounts to 2.3 to 3.0 millimetres, preferably 2.6 millimetres, the greatest diameter of the cover surface (2) amounts to 1.5 to 2.0 millimetres, preferably 1.7 millimetres and the distance between both end surfaces (1 or 2) amounts to 3.0 to 4.8 millimetres, preferably 4.0 millimetres.

8. Anvil prothesis according to one of the preceding claims, characterised thereby, that it additionnally displays at least one recess, for example a trough (14), a chamfer, a groove (15) and so forth, preferably in the region of both its end surfaces (1 or 2).

9. Anvil prothesis according to claim 1, characterised thereby, that the bio-active material is a compound material.

10. Anvil prothesis according to claim 1, characterised thereby, that the bio-active material consists of sinter products containing apatite.

11. Anvil prothesis according to claim 1, characterised thereby, that the bio-active material consists of glass-ceramic material.

12. Anvil prothesis according to claim 1, characterised thereby, that it consists of a core material with a coating of bio-active material.

13. Anvil prothesis according to at least one of the preceding claims, characterised thereby, that it additionally contains insoluble bio-inert material in at least one partial surface region.

14. Anvil prothesis according to claim 13, characterised thereby, that it displays in part a coating of insoluble bio-inert material.

15. Anvil prothesis according to claim 14, characterised thereby, that the coating consists of at least one additively applied protective layer (S(+)) of a thickness between 0.25 and 10 micrometres.

16. Anvil prothesis according to claim 14, characterised thereby, that the coating consists of at least one subtractively generated conversion layer (S(−)) of a thickness between 0.25 and 5 micrometres.

17. Anvil prothesis according to the claims 14 to 16, characterised thereby, that the coating consists of at least one conversion layer (S(−)) generated at the side of the prothesis cone and at least one additively applied protective layer (S(+)) resting on this.

18. Anvil prothesis according to at least one of the claims 13 to 17, characterised thereby, that it consists of insoluble bio-inert material at least in its shell region (3).

19. Anvil prothesis according to at least one of the claims 13 to 18, characterised thereby, that merely the end surfaces (base surface (1) and cover surface (2)) consist of bio-active material.

20. Anvil prothesis according to at least one of the claims 13 to 19, characterised thereby, that the bio-inert material or the bio-inert layer (S(+) or S(−)) consists of at least one of the following substances:

a) metals such as gold, platinum, titanium as well as metal alloys,

b) carbon in suitable modifications, such as pyrolytic carbon (graphite), carbon compounds such as silicon carbide (SiC), titanium carbide (TiC) and boron carbide ($B_4C$),

c) special ceramic materials such as hexagonal boron nitride (BN), titanium nitride (TiN) and silicon nitride ($Si_3N_4$),

d) partially crystalline inorganic compound systems such as enamels,

e) inorganic single-component glasses (for example flint glass) or multi-component glasses, and

f) oxides such as titanium dioxide ($TiO_2$), zirconium dioxide ($ZrO_2$) and aluminium oxide ($Al_2O_3$).

21. Anvil prothesis according to at least one of the claims 13 to 20, characterised thereby, that the bio-inert material or the bio-inert layer (S(−)) consists of a residual bioglass ceramic material or a residual bioceramic material or a bioglass, which is apatite-free and in a given case additionally displays a silane layer.

22. Process for the manufacture of an anvil prothesis according to at least one of the claims 13 to 21, characterised thereby, that the shell surface (3) of this anvil prothesis (4) is subjected under pinpointed substance supply to a surface-finishing treatment for the generation of at least one additively applied, permanently adhering, inorganic bio-inert protective layer (S(+)), which in vivo acts as biochemical barrier layer.

23. Process for the manufacture of an anvil prothesis according to at least one of the claims 13 to 21, characterised thereby, that the shell surface (3) of this anvil prothesis (4) is subjected under pinpointed substance removal or substance exchange to a chemical finishing treatment for the generation of at least one subtractively obtained, permanently adhering bio-inert conversion (leaching) layer (S(−)), which in vivo acts as biochemical barrier layer.

24. Process according to claim 22, characterised thereby, that the protective layer (S(+)) is preferably applied through at least one of the following steps:

a) galvanising or vapour-depositing,

b) sputtering,

c) precipitating out of organic solutions or vapourising under vacuum,

d) dipping, spraying or scattering-on with subsequent thermal treatment,

e) immersion in water glass with subsequent heating to about 400° C or immersion-glazing from single-component or multi-component smelt batches and

f) simultaneous evaporation or evaporation of the metals with subsequent oxidising treatment.

25. Process according to claim 23, characterised thereby, that the shell surface (3) to be coated is treated with aqueous acid solutions or with aqueous salt solutions of molecular concentrations between 0.001 and 0.1 for between 5 minutes and 3 hours at temperatures between 20 to 100° C.

26. Process according to claim 25, characterised thereby, that hydrochlorid acid (HCl) of a molecular concentration between 0.1 and 0.001 is used as aqueous acid solution.

27. Process according to claim 25, characterised thereby, that standard acetate buffer solution of a molecular concentration between 0.001 and 0.25 is used as aqueous salt solution.

28. Process according to claim 23, characterised thereby, that the coated parts (3) of the anvil prothesis (4) are subsequently subjected to a thermal sealing treatment and/or silanising treatment.

**Revendications**

1. Prothèse d'enclume pouvant être insérée entre l'étrier et le manche du marteau ou respectivement entre l'étrier et le manche du marteau et le tympan, caractérisée en ce que la prothèse d'enclume

a) présente une forme géométrique fermée (4) se rétrécissant, se composant de deux surfaces de fermeture configurées en une surface de base (1) et une surface supérieure (2) et d'une surface d'enveloppe (3) les reliant, avec, en plus les conditions qui suivent:

α) la surface de base (1) est plus grande que la surface supérieure (2),

β) le plus grand diamètre de la surface de base (1) est plus petit que la distance entre les deux surfaces de fermeture,

γ) le plus grand diamètre de la surface supérieure (2) est plus grand que le plus grand rayon de la surface de base (1); et

b) elle se compose d'un matériau bioactif.

2. Prothèse d'enclume selon la revendication 1, caractérisée en ce que les deux surfaces de fermeture (1 ou respectivement 2) ont des formes géométriques analogues, comme un cercle, une ellipse, un polygone régulier, un tracé polygonal irrégulier ou respectivement leur combinaison.

3. Prothése d'enclume selon l'une quelconque des revendications précédentes, caractérisée en ce que les formes géométriques des deux surfaces de fermeture (1 ou respectivement 2) ne sont pas analogues.

4. Prothèse d'enclume selon l'une quelconque des revendications précédentes, caractérisée en ce que les deux surfaces de fermeture (1 ou respectivement 2) se trouvent dans des plans parallèles.

5. Prothèse d'enclume selon l'une quelconque des revendications précédentes, caractérisée en ce que la ligne de jonction des centres géométriques (5 ou respectivement 6) des deux surfaces de fermeture (1 ou respectivement 2) leur est perpendiculaire.

6. Prothèse d'enclume selon l'une quelconque des revendications 1 et 3 à 5, caractérisée en ce que la forme spatiale (4) se compose d'un tronc de cône, dont deux segments symétriques en forme de paraboloïde ont été retirés le long de la surface d'enveloppe (3) de façon que les contours des surfaces de coupe en résultant présentent la forme d'une parabole (7 ou respectivement 8) reposant sur la trace de la surface supérieure (2) et s'ouvrant vers la surface de base (3).

7. Prothèse d'enclume selon l'une quelconque des revendications précédentes, caractérisée en ce que le plus grand diamètre de la surface de base (1) est de 2,3 à 3,0 mm, de préférence de 2,6 mm, le plus grand diamètre de la surface supérieure (2) est de 1,5 à 2,0 mm, de préférence de 1,7 mm, et la distance entre les deux surfaces de fermeture (1 ou respectivement 2) est de 3,0 à 4,8 mm, de préférence de 4,0 mm.

8. Prothèse d'enclume selon l'une quelconque des revendications précédentes, caractérisée en ce qu'elle présente en plus, avantageusement dans la zone de ses deux surfaces de fermeture (1 ou respectivement 2), au moins un évidement, par exemple une cavité (14), un chanfrein, une rainure (15) et analogues.

9. Prothèse d'enclume selon la revendication 1, caractérisée en ce que le matériau bioactif est un matériau composé.

10. Prothèse d'enclume selon la revendication 1, caractérisée en ce que le matériau bioactif se compose de produits frittés contenant de l'apatite.

11. Prothèse d'enclume selon la revendication 1, caractérisée en ce que le matériau bioactif se compose d'une vitrocéramique.

12. Prothèse d'enclume selon la revendication 1, caractérisée en ce qu'elle se compose d'un matériau de cœur avec une enduction d'un matériau bioactif.

13. Prothèse d'enclume selon l'une quelconque des revendications précédentes, caractérisée en ce qu'elle contient, de plus, dans au moins une zone partielle de sa surface, une matière bio-inerte insoluble.

14. Prothèse d'enclume selon la revendication 13, caractérisée en ce qu'elle présente partiellement une enduction d'une matière bio-inerte insoluble.

15. Prothèse d'enclume selon la revendication 14, caractérisée en ce que l'enduction se compose d'au moins une couche de protection (S(+)) formée par addition ayant une épaisseur comprise entre 0,25 et 10 μm.

16. Prothèse d'enclume selon la revendication 14, caractérisée en ce que l'enduction se compose d'au moins une couche de conversion produite par soustraction (S(−)) ayant une épaisseur comprise entre 0,5 et 5 μm.

17. Prothèse d'enclume selon l'une quelconque des revendications 14 à 16, caractérisée en ce que l'enduction se compose d'au moins une couche de conversion (S(−)) produite du côté noyau de la prothèse et, sur celle-ci, au moins une couche de protection (S(+)) formée par addition.

18. Prothèse d'enclume selon l'une quelconque des revendications 13 à 17, caractérisée en ce qu'elle se compose, au moins dans sa zone d'enveloppe (3), d'une matière bio-inerte insoluble.

19. Prothèse d'enclume selon l'une quelconque des revendications 13 à 18, caractérisée en ce que seules les surfaces de fermeture: surface de base (1) et surface supérieure (2) se composent d'une matière bioactive.

20. Prothèse d'enclume selon l'une quelconque des revendications 13 à 19, caractérisée en ce que la matière bio-inerte ou respectivement la couche bio-inerte (S(+) ou S(−)) se compose d'au moins l'une des substances qui suivent:

a) métaux, comme l'or, le platine, le titane ainsi qu'alliages de métaux;

b) carbone aux modifications appropriées, comme du carbone pyrolytique (graphite); des composés de carbone, comme le carbure de silicium (SiC), le carbure de titane (TiO), le carbure de bore (B₄C);

c) matières céramiques spéciales comme du nitrure de bore hexagonal (BN), du nitrure de titane (TiN), du nitrure de silicium (Si₃N₄);

d) systèmes composés anorganiques partiellement cristallins comme des émaux;

e) verres inorganiques à un composant (comme du quartz fondu) ou plusieurs composants;

f) oxydes, comme du bioxyde de titane ($TiO_2$), du bioxyde de zirconium ($ZrO_2$) et de l'oxyde d'aluminium ($Al_2O_3$).

21. Prothèse d'enclume selon l'une quelconque des revendications 13 à 20, caractérisée en ce que la matière bio-inerte ou respectivement la couche bio-inerte (S(−)) se compose d'un résidu sans apatite de biovitrocéramique ou biocéramique ou bioverre, qui le cas échéant peut de plus présenter une couche de silane.

22. Procédé de fabrication d'une prothèse d'enclume selon l'une quelconque des revendications 13 à 21, caractérisé en ce que la surface d'enveloppe (3) de cette prothèse d'enclume (4) est soumise, avec un apport approprié de matière, à un post-traitement de surface pour la production d'au moins une couche de protection bio-inerte (S(+)) formée par addition, fixée de manière durable, inorganique, et agissant in vivo comme couche biochimique de barrage.

23. Procédé de fabrication d'une prothèse d'enclume selon l'une quelconque des revendications 13 à 21, caractérisé en ce que la surface d'enveloppe (3) de cette prothèse d'enclume (4) est soumise, avec enlèvement approprié de matière ou respectivement échange de matière, à un post-traitement chimique pour la production d'au moins une couche de conversion (d'extraction) (S(−)) bio-inerte obtenue par soustraction, fixe en permanence, et agissant in vivo comme couche biochimique d'arrêt.

24. Procédé selon la revendication 22, caractérisé en ce que la couche de protection (S(+)) est avantageusement produite par au moins une des étapes qui suivent:

a) galvanisation ou métallisation au vide;

b) pulvérisation;

c) dépôt de solutions organiques ou métalliques sous vide;

d) immersion, pulvérisation ou saupoudrage avec traitement thermique subséquent;

e) immersion dans du silicate de potassium avec ensuite chauffage à environ 400° C ou respectivement vitrification par immersion dans des mélanges en fusion à un ou plusieurs composants;

f) métallisation sous vide simultanée ou métallisation des métaux avec traitement subséquent d'oxydation.

25. Procédé selon la revendication 23, caractérisé en ce que la surface d'enveloppe (3) à enduire est traitée avec des solutions aqueuses acides ou avec des solutions aqueuses de sel ayant des normalités comprises entre 0,01 et 0,1 entre 5 minutes et 3 heures à des températures comprises entre 20 et 100° C.

26. Procédé selon la revendication 25, caractérisé en ce que l'on utilise, comme solution aqueuse acide, de l'acide chlorhydrique (HCl) à 0,1–0,001 N.

27. Procédé selon la revendication 25, caractérisé en ce qu'on utilise, comme solution aqueuse de sel, une solution standard de tampons d'acétates à 0,001–0,25 N.

28. Procédé selon la revendication 23, caractérisé en ce que les parties enduites (3) de la prothèse d'enclume (4) sont ensuite soumises à un traitement thermique de vitrification et/ou silanisation.

0064278

Fig. 1

Fig. 2a

Fig. 2b

Fig. 3

Fig. 4

Fig. 5

11